# EUROPEAN PATENT APPLICATION

(11) **EP 4 458 432 A1**
(43) Date of publication of application: **06.11.2024**
(21) Application number: 22916428.0
(22) Date of filing: 17.11.2022
(51) Int. Cl.: A63B 24/00, A63B 71/06, G06T 13/40, G06T 19/20

(54) **METHOD AND APPARATUS FOR PROVIDING WORKOUT GUIDE VIDEO**

(30) Priority: 28.12.2021 KR 20210190372; 10.05.2022 KR 20220057272
(71) Applicant: DRAX Inc., Anyang-si, Gyeonggi-do 14086 (KR)
(72) Inventor: YOO, Seon Kyung, Seoul 08251 (KR); PARK, Jae Sang, Seongnam-si, Gyeonggi-do 13626 (KR)
(74) Representative: Bianchetti & Minoja with Trevisan & Cuonzo IPS SRL
(86) International application number: PCT/KR2022/018236
(87) International publication number: WO 2023/128274

(57) **Abstract**

Disclosed are a method of providing an exercise guide video and an apparatus therefor. The method of providing an exercise guide video includes recognizing a posture of a user while the exercise guide video is being displayed, comparing the posture of the user with a reference posture included in the exercise guide video and corresponding to the posture of the user, and when, during a certain time period, a degree of similarity between the posture of the user and the reference posture is less than a reference value, ending the displaying of the exercise guide video.

## Description

### Technical Field

The present disclosure relates to a method and an apparatus for providing an exercise guide video.

### Background Art

Recently, as work-life balance, which indicates the balance of work with one's personal life, has drawn increased attention, interest in one's own life, such as exercise, after work, during the weekend, or during free time has increased. As part of work-life balance, users' desire to manage their health has increased, and to improve one's quality of life by investing a small amount of time, more and more users have been engaging in various exercise activities, such as swimming, gym workout, personal training (PT), golf, Pilates, etc.

As the number of users exercising has increased, the desire to exercise with the correct posture has also increased, and thus, videos, etc. providing exercise guides have been introduced. Exercise guide videos show instructors, trainers, etc. performing aerobic motions, yoga motions, or health workout motions, and users may exercise by following the motions of the instructors, etc. shown in the exercise guide videos.

However, previous exercise guide videos may only guide exercises in units of time and does not selectively guide each user.

### Disclosure

### Technical Problem

The present disclosure provides a method and an apparatus for providing an exercise guide video according to a level of a user.

The present disclosure provides a method and an apparatus for providing an exercise guide video based on reverse playing.

The present disclosure provides a method and an apparatus for providing an exercise guide video based on a motion of a user.

### Technical Solution

According to an aspect of the present disclosure, there is provided a method of providing an exercise guide video, the method including: displaying an exercise guide video including repetitive motions; recognizing a posture of a user while the exercise guide video is being displayed; comparing the posture of the user with a reference posture included in the exercise guide video and corresponding to the posture of the user; and when, during a certain time period, a degree of similarity between the posture of the user and the reference posture is less than a reference value, ending the displaying of the exercise guide video.

Also, the certain time period may be greater than or equal to a time period corresponding to one set motion included in the exercise guide video.

Also, the method may further include, when the degree of similarity between the posture of the user and the reference posture is greater than or equal to the reference value, displaying the exercise guide video according to an exercise speed of the user.

Also, the displaying of the exercise guide video may include, when it is determined that the posture of the user is ahead of the reference posture displayed in the exercise guide video by a reference section, increasing a play speed of the exercise guide video such that the displayed reference posture corresponds to the posture of the user.

Also, the displaying of the exercise guide video may include, when it is determined that the posture of the user is behind the reference posture displayed in the exercise guide video by a reference section, decreasing a play speed of the exercise guide video such that the displayed reference posture corresponds to the posture of the user.

Also, the displaying of the exercise guide video may include, when it is determined that the posture of the user is behind the reference posture displayed in the exercise guide video by a reference section, pausing the displaying of the exercise guide video until the displayed reference posture corresponds to the posture of the user.

Also, a reference section may be greater than or equal to a section corresponding to a sub-set motion in the exercise guide video.

Also, the method may further include: when, during the certain time period, the degree of similarity between the posture of the user and the reference posture is greater than or equal to the reference value, changing, according to the degree of similarity, a body shape of a character indicating the user; and displaying the changed body shape of the character.

Also, the method may further include generating the exercise guide video including two or more set motions based on reverse playing of some sections of an original video including one set motion.

An electronic device according to an embodiment includes: a display configured to display an exercise guide video including repetitive motions; a motion sensing sensor configured to recognize a posture of a user; and a processor configured to compare the posture of the user with a reference posture included in the exercise guide video and corresponding to the posture of the user and when, during a certain time period, a degree of similarity between the posture of the user and the reference posture is less than a reference value, end the displaying of the exercise guide video.

Also, the electronic device may further include a film disposed on the display and configured to transmit light corresponding to the exercise guide video and reflect light corresponding to the posture of the user. The certain time period may be greater than or equal to a time period corresponding to one set motion included in the exercise guide video.

Also, the processor may further be configured to, when the degree of similarity between the posture of the user and the reference posture is greater than or equal to the reference value, display the exercise guide video according to an exercise speed of the user.

Also, the processor may further be configured to, when it is determined that the posture of the user is ahead of the reference posture displayed in the exercise guide video by a reference section, increase a play speed of the exercise guide video such that the displayed reference posture corresponds to the posture of the user.

Also, the processor may further be configured to, when it is determined that the posture of the user is behind the reference posture displayed in the exercise guide video by a reference section, decrease a play speed of the exercise guide video such that the displayed reference posture corresponds to the posture of the user.

Also, the processor may further be configured to, when it is determined that the posture of the user is behind the reference posture displayed in the exercise guide video by a reference section, pause the displaying of the exercise guide video until the displayed reference posture corresponds to the posture of the user.

Also, a reference section may be greater than or equal to a section corresponding to a sub-set motion in the exercise guide video.

Also, the processor may further be configured to: when, during the certain time period, the degree of similarity between the posture of the user and the reference posture is greater than or equal to the reference value, change, according to the degree of similarity, a body shape of a character indicating the user; and display the changed body shape of the character.

### Advantageous Effects

An exercise guide video according to an embodiment may adjust the number of times of a set motion.

An exercise guide video according to an embodiment may increase a user's sense of immersion with respect to an exercise by providing a seamless image of a set motion which is repetitive motions.

An exercise guide video according to an embodiment may be adjusted by an exercise speed of a user thus to be relatively more usefully provided to the user.

### Description of Drawings

FIG. 1 is a schematic diagram showing an exercise machine management system according to an embodiment.
FIG. 2 is a block diagram showing a management server according to an embodiment.
FIG. 3 is a block diagram showing an exercise machine according to an embodiment.
FIG. 4 is a block diagram showing a mirror display according to an embodiment.
FIG. 5 is a flowchart for describing a method of providing an exercise guide video, according to an embodiment.
FIG. 6 is a diagram of an original video composed of a set motion, according to an embodiment.
FIG. 7 is a reference diagram for describing a method of generating an exercise guide video including a plurality of set motions, according to an embodiment.
FIG. 8 is a reference diagram for describing a method of generating an exercise guide video including a plurality of set motions, according to another embodiment.
FIG. 9 is a reference diagram for describing a method of providing a video including a plurality of set motions, according to a comparative embodiment.
FIG. 10 is a diagram of an original video including a plurality of set motions, according to an embodiment.
FIG. 11 is a reference diagram for describing a method of providing an exercise guide video by using one set section from among a plurality of set sections, according to an embodiment.
FIG. 12 is a reference diagram for describing a method of providing an exercise guide video by using a plurality of set sections, according to an embodiment.
FIG. 13 is a diagram of an original video including different types of set motions, according to an embodiment.
FIG. 14 is a reference diagram for describing a method of generating an exercise guide video including repetitive exercises, according to an embodiment.
FIG. 15 is a reference diagram for describing a method of generating an exercise guide video including repetitive exercises, according to another embodiment.
FIG. 16 is a flowchart of a method of providing an exercise guide video based on a user's motion, according to an embodiment.
FIG. 17 is a reference diagram for describing a method of providing an exercise guide video, when a user's posture is ahead of a reference posture, according to an embodiment.
FIG. 18 is a reference diagram for describing a method of providing an exercise guide video, when a user's posture is behind a reference posture, according to an embodiment.
FIG. 19 is a reference diagram for describing a method of providing an exercise guide video, when a user's posture is behind a reference posture, according to another embodiment.
FIG. 20 is a reference diagram for describing a method of displaying a character referring to a user, according to an embodiment.
FIG. 21 is a reference diagram for describing a method of indicating an exercise effect by using a character, according to an embodiment.

### Mode for Invention

In the present disclosure, general terms that have been widely used nowadays are selected, when possible, in consideration of functions of the present disclosure, but non-general terms may be selected according to the intentions of technicians in the this art, precedents, or new technologies, etc. Also, some terms may be arbitrarily chosen by the present applicant. In this case, the meanings of these terms will be explained in corresponding parts of the disclosure in detail. Thus, the terms used herein should be defined not based on the names thereof but based on the meanings thereof and the whole context of the present disclosure.

The terms, such as "first," "second," etc., may be used to describe various components, but the components shall not be limited by the terms. These terms are only used to distinguish one element from another.

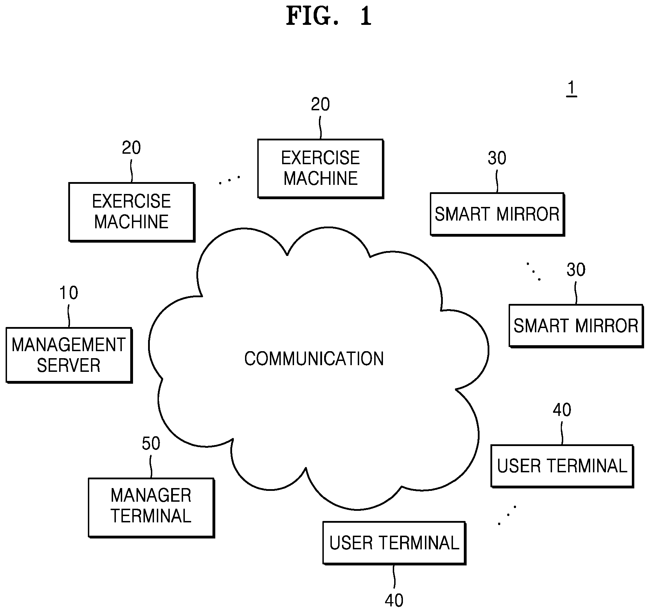

FIG. 1 is a schematic diagram showing an exercise machine management system 1 according to an embodiment. Referring to FIG. 1, the system 1 may include a management server 10, an exercise machine 20, a mirror display 30, a user terminal 40, and a manager terminal 50 connected in a network. In addition, the system 1 may further include a payment server, a contents server, etc. linked with the management server 10.

The network may be realized as various types of wired or wireless networks, such as a local area network (LAN), a wide area network (WAN), a value added network (VAN), a personal area network (PAN), a mobile radio communication network, or a satellite communication network.

The management server 10 may be connected with the exercise machine 20, the mirror display 30, the user terminal 40, the manager terminal 50, etc. through the network and capable of data transmission and reception thereto and therefrom, may store an exercise schedule, an exercise item list, etc. of a user in response to a request of the manager terminal 50, may store an exercise result, etc. of the user from information received from the user terminal 40 or the exercise machine 20, and may provide the exercise result to the manager terminal 50 or the user terminal 40 in response to a request of the manager terminal 50 or the user terminal 40.

Also, the management server 10 may control the exercise machine 20, etc. in a health center in response to a request of the user terminal 40 or may use an exercise result received from the exercise machine 20 to generate calorie information, etc. corresponding to an exercise history or the exercise result.

The management server 10 may be an operating server of an Internet site. The management server 10 may operate an exclusive application and/or web program connected to the exercise machine 20, the mirror display 30, the user terminal 40, the manager terminal 50, etc. or may perform a function of supporting the exercise machine 20, the mirror display 30, the user terminal 40, the manager terminal 50, etc. Also, the management server 10 may perform a function of supporting an interface necessary for establishing, on the web/application, an application such as an application programming interface (API), thereby driving an application for realizing a content platform of a digital information display.

The exercise machine 20 may be realized as a weight training machine, a running machine, or the like and may include an exercise body moving according to a movement of a user. Also, the exercise machine 20 may provide an exercise guide to the user under control by the management server 10 and may transmit an exercise result of the user to the management server 10. The exercise machine 20 may be set in various modes according to a manipulation of the user or under control by the management server 10.

The user terminal 40 may be registered in the management server 10 to receive an exercise management service and may be configured to transmit and receive data by accessing the management server 10, the entrance terminal 20, the exercise machine 20, etc. through a wired or wireless network. The user terminal 40 may include a smartphone, a personal computer (PC), a tablet PC, a notebook computer, a smart television (TV), a cellular phone, a personal digital assistant (PDA), a laptop, a media player, a global positioning system (GPS) device, an electronic book reader, a digital broadcasting terminal, a navigation device, a digital camera, a wearable device and other mobile or immobile computing devices, and a smart band. However, the present disclosure is not limited thereto. Here, the user terminal 40 may denote not only a terminal owned by a user receiving an exercise management service, but may also denote a predetermined terminal through which the registered user may access the management server 10 and perform a login operation.

The manager terminal 50 may be registered in the management server 10 to provide an exercise management service and may be configured to transmit and receive data by accessing the management server 10 through a wired or wireless network. The manager terminal 50 may include a smartphone, a PC, a tablet PC, a notebook computer, a smart TV, a cellular phone, a PDA, a laptop, a media player, a GPS device, an electronic book reader, a digital broadcasting terminal, a navigation device, a digital camera, and a wearable device and other mobile or immobile computing devices. However, the present disclosure is not limited thereto. Here, the manager terminal 50 may denote not only a terminal owned by a manager registered in the management server 10, but may also denote a predetermined terminal through which the registered manager may access the management server 10 and perform a login operation.

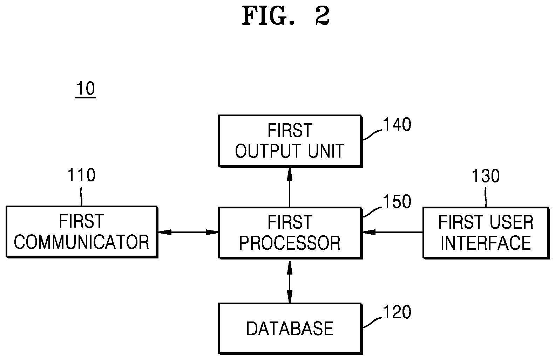

FIG. 2 is a block diagram showing the management server 10 according to an embodiment. Referring to FIG. 2, the management server 10 may include a first communicator 110 communicating with an external terminal, for example, the entrance terminal 20, the exercise machine 20, the mirror display 30, the user terminal 40, the manager terminal 50, etc. through a network, a database 120 storing information about a user's exercise program, a user's exercise history, an entrance list of a health center, a mode of the exercise machine 20, etc., a first user interface 130 receiving a user command of a manager, etc. managing the management server 10, a first output unit 140 displaying or notifying a state of the heath center, and a first processor 150 performing general functions of the management server 20. The first processor 150 may generate the user's exercise program, manage a use situation of the center, or set the mode of the exercise machine 20 in response to a request by the manager terminal 50.

Although not shown in the drawing, the entrance terminal 20, the manager terminal 50, and the user terminal 40 may also include a communicator, a storage, a user interface, a processor, etc.

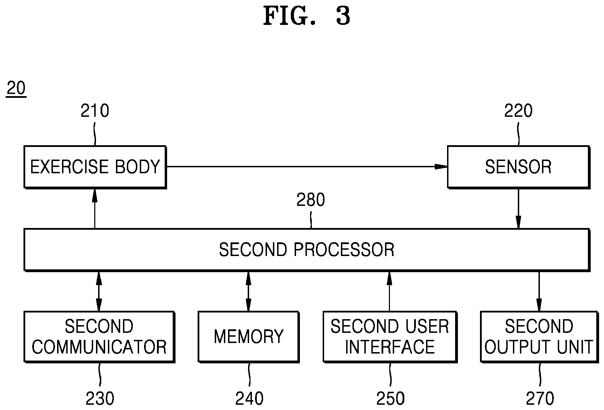

FIG. 3 is a block diagram showing the exercise machine 20 according to an embodiment. The exercise machine 20 according to an embodiment may include an aerobic exercise machine or an anaerobic exercise machine. For example, the exercise machine 20 may include aerobic exercise machines, such as a treadmill, a bicycle, an elliptical trainer, a stair exercise machine, etc., or anaerobic exercise machines, such as a chest press machine, a shoulder press machine, an arm curl machine, a lat pull down machine, a pec deck fly machine, a seated row machine, a long pull machine, a chinning dipping machine, a leg curl machine, a leg extension machine, a leg press machine, an inner thigh machine, an outer thigh machine, a total him machine, a torso back extension machine, an abdominal machine, etc.

The exercise machine 20 may include an exercise body 210, a sensor 220, a second communicator 240, a first memory 250, a second user interface 260, a second output unit 270, and a second processor 280.

The exercise body 210 is a physical exercise machine configured to move according to a movement of a user. The exercise body 210 according to an example may add, decrease, or maintain a load according to a configured exercise level. The exercise body 210 may vary according to a type of the exercise machine 20.

The sensor 220 may sense movements of components included in the exercise body 210 and may transmit a result of the sensing to the second processor 280. The sensor 220 may include a laser sensor 220, a motion sensor, a gyro sensor, etc.

The second communicator 240 may communicate with an external device, for example, the management server 10, the entrance terminal 20, another exercise machine 20, the user terminal 40, the manager terminal 50, etc. The second communicator 240 may communicate with the management server 10 through various types of wired or wireless networks, such as a LAN, a WAN, a VAN, a PAN, a mobile radio communication network, a satellite communication network, etc. Also, the second communicator 240 may communicate with the entrance terminal 20, the user terminal 40, and the manager terminal 50 through a short-range wireless communication network, such as a LAN, a PAN, etc. By doing so, the exercise machine 20 may be prevented from being controlled by other external devices, except for the management server 10. However, it is not limited thereto. According to cases, the second communicator 240 may remotely communicate with the manager terminal 50.

The first memory 250 may store various information used by at least one component (for example, the second processor 280 or the sensor 220) included in the exercise machine 20. The information may include, for example, software (for example, a program), and input data or output data with respect to a command related to the software. The first memory 250 may include a first volatile memory 250 or a first nonvolatile memory 250. For example, the first memory 250 may include an adjustable exercise range or a threshold exercise range predetermined according to user information.

The second user interface 250 may receive a command or data to be used by the exercise machine 20 from the outside (for example, a user) of the exercise machine 20. The second user interface 250 may include, for example, a microphone, a mouse, a keyboard, an electronic tag, or a digital pen (for example, a stylus pen). For example, the user may input user information through the second user interface 250. For example, the user information may include one or more from among a user's name, age, gender, height, weight, and exercise history.

The second output unit 270 may provide output information. The output information according to an example may be the user information, user's exercise information, or information for guiding a user's exercise. For example, the second output unit 270 may include a display for displaying the output information. Also, the second output unit 270 may be physically or electrically connected to the exercise body 210. For example, the display may be mounted on a frame structure of the exercise body 210. However, the present disclosure is not limited thereto, and the display 60 may include a second communicator 230 and may be connected with the exercise body 210 to receive a predetermined signal from the exercise body 210, even when the display 60 is arranged to be apart from the exercise body 210.

The second processor 280 may generally control the exercise machine 20 and may control the second communicator 240 to perform communication with an external device. The second processor 280 may execute, for example, software (for example, a program) to control at least another component (for example, a hardware or a software component) connected with the second processor 280 and to perform various data processing or calculating operations. According to an embodiment, at least as part of the data processing or calculating operations, the second processor 280 may load a command or data received from another component (for example, the sensor 220 or the second communicator 230) in the first volatile memory of the first memory 250, process the command or data stored in the volatile memory, and store resultant data in the nonvolatile memory of the first memory 250.

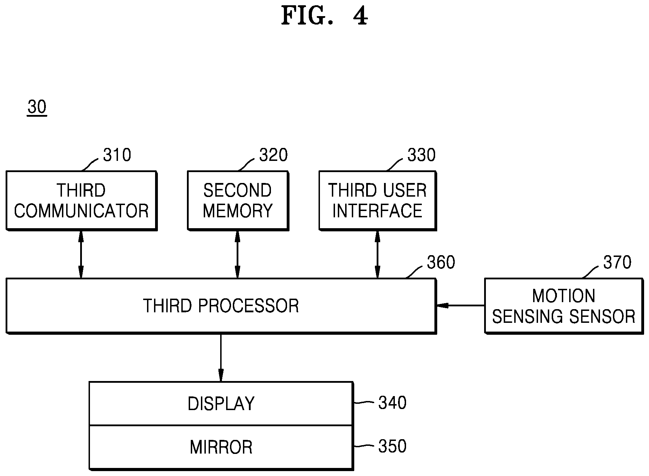

FIG. 4 is a block diagram showing the mirror display 30 according to an embodiment. The mirror display 30 may be configured to provide at least one of a mirror function and a display function according to necessity of a user. The mirror display 30 may further include a film (not shown) including a polarization function on the display, and thus, may be understood to encompass generally known devices capable of simultaneously performing a function of a display and a function of a mirror by reflecting certain light (for example, light corresponding to a posture of a user) and transmitting certain light (for example, light corresponding to an image output from the display).

The mirror display 30 may include a third communicator 310, a second memory 320, a third user interface 330, a display 340, a mirror 350, and a third processor 360.

The third communicator 310 may communicate with an external device, for example, the management server 10, the user terminal 40, the manager terminal 50, etc. The third communicator 310 may communicate with the management server 10 through various types of wired or wireless networks, such as a LAN, a WAN, a VAN, a PAN, a mobile radio communication network, a satellite communication network, etc. Also, the third communicator 310 may communicate with the user terminal 40 and the manager terminal 50 through a short-range wireless communication network, such as a LAN, a PAN, etc. By doing so, the mirror display 30 may be prevented from being controlled by other external devices, except for the management server 10. However, it is not limited thereto. According to cases, the third communicator 310 may remotely communicate with the manager terminal 50.

The second memory 320 may store various information used by at least one component (for example, the third processor 360) included in the mirror display 30. The information may include, for example, software (for example, a program) and input data or output data with respect to a command related to the software. The second memory 320 may include a second volatile memory 320 or a second nonvolatile memory 320. For example, the second memory 320 may pre-store an original video which may be used to generate an exercise guide video.

The third user interface 250 may receive various user commands. For example, the third user interface may receive a user command for playing an exercise guide video which may be displayed on the mirror display 30. A user may input, through the third user interface, an exercise item, the number of sets, etc. with respect to which the exercise guide video is to be played.

The display may display the exercise guide video under control by the third processor 360. The display may be realized as a liquid-crystal display (LCD), an organic light-emitting diode (OLED), etc. and may also perform a function of the third user interface by further including a touch screen.

Also, the mirror 350 may be arranged on at least a portion of the entire surface of the display. The mirror 350 may include a film, for example, a polarization film, reflecting light incident from the outside and transmitting light incident from the display.

The third processor 360 may generally control the mirror display 30 and may control the third communicator 310 to perform communication with an external device. The third processor 360 may execute, for example, software (for example, a program) to control at least another component (for example, a hardware or software component) connected with the third processor 360 and to perform various data processing or calculating operations. According to an embodiment, at least as part of the data processing or calculating operations, the third processor 360 may load a command or data received from another component (for example, the third communicator 230 or the third user interface) in the second volatile memory of the second memory 320, process the command or data stored in the second volatile memory, and store resultant data in the second nonvolatile memory of the second memory. For example, the third processor 360 may store an original video including a set motion received from the server, etc. in the second memory.

The original video may be divided into a plurality of sections. In detail, the original video may be divided into a preparation section R indicating a preparation motion, a finishing section E indicating a finishing motion, and a set section S which may indicate repetitive motions. For example, when the original video includes a squat exercise, which is an example of a set motion, the original video may be divided into the preparation section R indicating a motion of preparing the squat exercise, a finishing section E indicating a motion of finishing the squat exercise, a set section S including repetitive squat motions, that is, the set section S indicating a motion of sitting down and standing up.

The set section S may be divided into two sub-sections with respect to an inflection point, for example, a point at which a direction of the motion is switched. When the set section S indicates the squat motion, the set section S may be divided into a first section indicating a sitting-down motion and a second section indicating a standing-up motion with respect to a sitting point of the squat motion. When the first section indicating the sitting-down motion is a forward-direction section F, the second section indicating the standing-up motion, which is a backward direction motion of the sitting-down motion, may be referred to as a backward-direction section B.

The third processor 360 may receive and store the original video divided into the plurality of sections from the outside, for example, a server. However, the third processor 360 is not limited thereto. The third processor 360 may store the received original video by dividing the received original video into the plurality of sections as described above. Alternatively, after the third processor 360 stores the original video, the third processor 360 may generate the exercise guide video by dividing the original video into the plurality of sections when the third processor 360 is requested to play the exercise guide video according to a user command, etc.

The mirror display 30 may further include a sensing sensor. The motion sensing sensor may be equipment for obtaining a user's motion and may include a vision camera, a vision sensor, an acceleration sensor, a gyro sensor, a geomagnetic sensor, an infrared sensor, etc.

The motion sensing sensor 370 may extract an area corresponding to a joint of a user from detected data and may obtain coordinate data, speed data, etc. of the corresponding joint.

In FIG. 4, the motion sensing sensor is described as a component of the mirror display, but the motion sensing sensor is not limited thereto. The motion sensing sensor may be a separate device. Alternatively, a camera of the user terminal may perform the function of the motion sensing sensor.

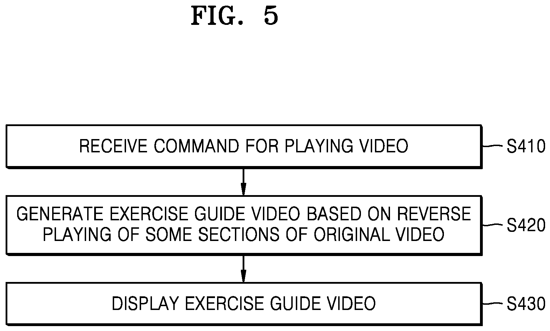

FIG. 5 is a flowchart for describing a method of providing an exercise guide video, according to an embodiment.

The third processor 360 may receive, from the third communicator 310, the third user interface 330, or the like, a command for playing an exercise guide video, the command including the number of times of set motions, in operation S410. When a user enters a predetermined distance from the mirror display 30, the mirror display 30 may be switched from a stand-by mode to an operation mode. Also, the mirror display 30 may request an exercise item to be played, from a user terminal, etc. carried by the user. The user terminal may transmit a pre-stored exercise item and the number of times of a set motion of the exercise item to the mirror display 30 to request the mirror display 30 to play the exercise guide video with respect to the corresponding exercise item. Alternatively, the user may input the exercise item and the number of times of the set motion through a user interface of the mirror display 30 so as to input a command for playing the exercise guide video.

The third processor 360 may generate the exercise guide video including the set motion corresponding to the number of times based on reverse playing of some sections of the original video, in operation S420. The third processor 360 may a video corresponding to the set motion by playing and reversely playing some sections of the original video. As the number of times of playing and reverse playing of some sections increases, the number of times of the set motion included in the generated video may also increase. A detailed method of generating the exercise guide video will be described below.

The third processor 360 may display the generated exercise guide video on the display 340 in operation S430. The third processor 360 may display the exercise guide video after the third processor 360 completes the generation of the exercise guide video or may simultaneously generate and display the exercise guide video. The generating and the displaying of the exercise guide video may be referred to as playing of the exercise guide video.

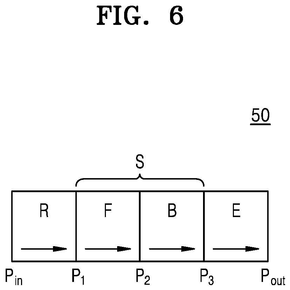

FIG. 6 is a diagram of an original video composed of a set motion, according to an embodiment. Referring to FIG. 6, an original video 50 may be divided into a plurality of different sections. The original video 50 may be divided into a preparation section R indicating a preparation motion, a finishing section E indicating a finishing motion, and a set section S which may indicate repetitive motions.

The set section S may be divided into a forward direction section F and a backward direction section B with respect to an inflection point of a motion, that is, a point at which a direction of the motion is switched. For example, the set section S indicating a motion of sitting down and then standing up in a squat exercise may be divided into the forward direction section F indicating a sitting-down motion from a start point of the set section S to a sitting point and the backward direction section B indicating a standing-up motion from the sitting point to an end point of the set section S. The motion included in the forward direction section F may be the opposite to the motion included in the backward direction section B. For example, the motion included in the forward direction video may be the sitting-down motion, while the motion included in the backward direction video may be the standing-up motion.

A time in which the forward direction section F is played may be the same as a time in which the backward direction section B is played, but it is not limited thereto. For example, a difference between the time in which the forward direction section F is played and the time in which the backward direction section B is played may be within an error range of 10 %.

A posture in the video at the start point of the set section S and a posture in the video at the end point of the set section S may be the same as each other. However, it is not limited thereto. For example, a difference between the posture in the start point of the set section S and the posture in the end point of set section S may be within an error range of 10 %.

The original video 50 may be pre-stored in the second memory 320 of the mirror display 30.

The third processor 360 may generate an exercise guide video 50a including a plurality of repetitive motions, that is, a plurality of set motions, by using the original video 50 of the single set motion of FIG. 5.

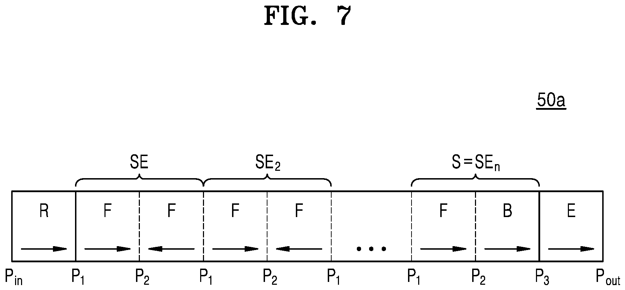

FIG. 7 is a reference diagram for describing a method of generating the exercise guide video 50a including a plurality of set motions, according to an embodiment.

When a command for playing an exercise guide video including n times set motions is received from the third user interface 330 or an external device, the third processor 360 may read the original video 50 corresponding to the exercise guide video from the second memory 320. The third processor 360 may temporarily store the original video 50 in the second memory 320 by receiving the original video 50 from an external device, for example, the management server 10, the user terminal 30, etc., and then may use the original video 50.

The original video 50 may include the plurality of sections R, F, B, and E as described with reference to FIG. 6, and the plurality of sections R, F, B, and E may be divided from one another by markers, etc. However, the original video 50 is not limited thereto. When the original video 50 is not divided into the plurality of sections R, F, B, and E, the third processor 360 may divide the original video 50 into the plurality of sections as described with reference to FIG. 6, by using markers, etc.

The third processor 360 may generate the exercise guide video 50a including n set motions based on reverse playing of some sections of the original video.

As illustrated in FIG. 7, the third processor 360 may play the preparation section R in the original video 50.

When playing the set section S, the third processor 360 may reversely play some sections of the set section S. In detail, because the forward direction section F includes the sitting-down motion, the reverse playing of the forward direction section F may include the standing-up motion. Accordingly, after the third processor 360 plays the forward direction section F, the third processor 360 may reversely play the forward direction section F, so as to generate a first set section S1 including the sitting-down and then stand-up motion. When the third processor 360 performs the operation of playing the forward direction section F and then reversely playing the forward direction section F n-1 times, n-1 set sections SE₁, SE₂, and SEₙ may be generated.

Also, the third processor 360 may generate the n^{th} set section SEn by sequentially playing the forward direction section F and the backward direction section B. Lastly, the third processor 360 may complete the generation of the exercise guide video 50a by playing the finishing section E.

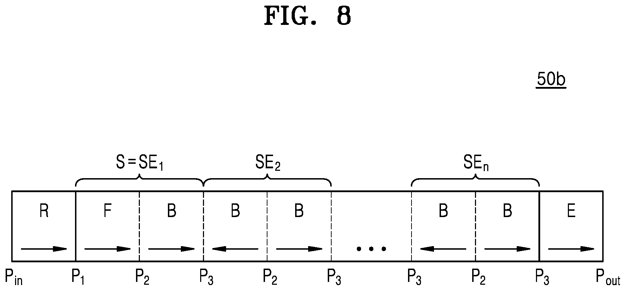

FIG. 8 is a reference diagram for describing a method of generating an exercise guide video including a plurality of set motions, according to another embodiment.

When the third processor 360 receives, from the third user interface 330, an external device, or the like, a request for playing an exercise guide video including n times set motions, the third processor 360 may generate the exercise guide video including the n set motions based on reverse playing of some sections of the original video 50.

As illustrated in FIG. 8, the third processor 360 may play the preparation section R in the original video 50.

The third processor 360 may generate a first set section SE₁ by playing the forward direction section F and the backward direction section B.

Also, the third processor 360 may generate a second set section SE₂ by reversely playing the backward direction section B and then playing the backward direction section B. For example, when the video includes the backward direction section B including a standing-up motion, the video including a sitting-down motion may be played by reversely playing the backward direction section B. The third processor 360 may generate n-1 times set sections SE₁ and SE₂, SEₙ by performing the operation of reversely playing the backward direction section B and then playing the backward direction section B n-1 times.

The third processor 360 may complete the generation of an exercise guide video 50b by playing the finishing section E.

As described above, the exercise guide videos 50a and 50b including the plurality of set motions may be generated based on the reverse playing of some sections of the original video 50 including a single set motion. Thus, the exercise guide video including seamless images may be generated. Because the original video indicating the single set motion is used, video without deviations of motions may be provided even when the set motion is repeated. When an exercise guide video including a plurality of set motions is obtained by photographing, a person repeating a set motion, for example, a trainer, may have a changed posture, as the set motion is repeated. However, the number of times of the set motion is increased by repeating the operation of playing and reverse playing of some sections of the original video, deviations of the motion may be basically prevented.

Also, there is no need to manufacture and store the original video for each time of the set motion, manufacturing costs of the original video may be reduced and the storage capacity of the original video may be decreased.

However, the plurality of set motions may be realized by repeatedly playing the set section of the original video 50 indicating a single set motion.

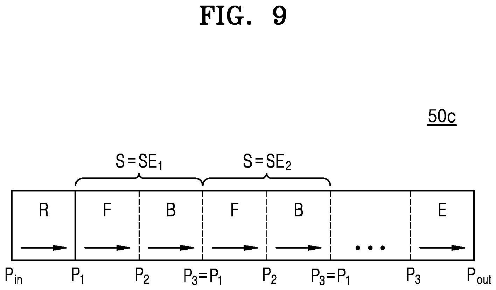

FIG. 9 is a reference diagram for describing a method of providing a video including a plurality of set motions, according to a comparative embodiment.

Referring to FIG. 9, the third processor 360 may play a set section SE₁ after playing the preparation section R of the original video 50. The third processor 360 may repeatedly play the set section S n times. That is, when, after the third processor 360 repeatedly plays the set section S=SE₁, an end point P₃ of the set section S=SE₁ is reached, the third processor 360 may move to a start point P₁ of the set section S and may replay a set section S=SE₂.

The repeated playing includes moving to the start point P₁ of the set section S from the end point P₃ of the set section S, and thus, even though it may be a short period of time, disconnection of the video may occur. Also, when a posture in the video at the end point P₃ of the set section S does not correspond to a posture in the video at the start point P₁ of the set section S, the degree of disconnection of the video may further be increased.

The original video 50 may be generally obtained by photographing an exercise process of a human being, and thus, the posture at the end point P₃ of the set section S and the posture at the start point P₁ of the set section S may not completely correspond to each other. Thus, it is expected that disconnection may inevitably occur in an exercise guide video 50C which is generated based on the repetitive playing. The disconnection phenomenon may deteriorate the user's sense of immersion with respect to the exercise.

However, according to an embodiment, when the exercise guide videos 50a and 50b are generated to include the plurality of set motions by using the reverse playing of some sections of the original video 50, the start point of the set motion and the end point of the set motion may be the same as each other, and thus, disconnection of the video may not occur. Thus, the user may watch the video with improved sense of immersion with respect to the exercise.

The original video 50 of FIG. 6 may be composed of one set motion. However, it is not limited thereto. The original video may include two or more set motions. Generally, a repetitive exercise may include a plurality of set motions. Thus, the original video may be composed of a basic number of times of the set motion, and the third processor 360 may generate an exercise guide video including the number of times of the set motion, which is greater than the basic number of times, based on reverse playing of some sections of the original video.

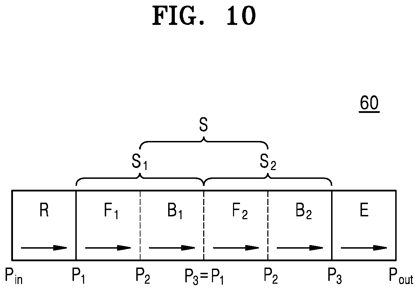

FIG. 10 is a diagram of an original video 60 including a plurality of set motions, according to an embodiment. As illustrated in FIG. 10, the original video 60 may include a set motion, the basic number of ties of which is two. For example, the set section S of the original video 60 may include a first set section S₁ and a second set section S₂ including the same type of set motions.

When an exercise guide video is to be generated, which includes the set motion, the number of times of which is greater than the basic number of times, the third processor 360 may increase the number of times of the set motion by repeating playing and reverse playing of some sections of the set section S.

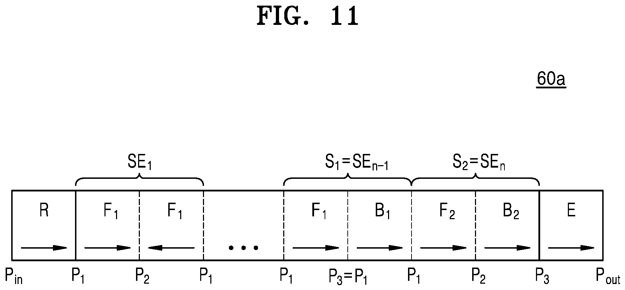

FIG. 11 is a reference diagram for describing a method of providing an exercise guide video by using one set section from among a plurality of set sections, according to an embodiment.

As illustrated in FIG. 11, for example, the third processor 360 may generate a first set section SE₁ by playing and reversely playing a forward direction section F₁ of a first set section S₁ of the set section S. The third processor 360 may generate n-2 times set sections by performing, n-2 times, the playing and the reverse playing of the forward direction section F₁ of the first set section S₁ of the set section S.

Also, the third processor 360 may generate an n-1^{th} set section SEn-₁ and an n^{th} set section SE_{N} by playing the first set section S1 and a second set section S2 of the original video 60.

In FIG. 11, the set sections are increased by playing and reversely playing the forward direction section F₁ of the first set section S₁. However, the set sections are not limited thereto. The set sections may be increased by playing and reversely playing a backward direction section B₁ of the first set section S₁. Alternatively, the number of times of the set motion may be increased by using playing and reverse playing of a forward direction section F₂ or a backward direction section B₂ of the second set section S₂.

Alternatively, the third processor 360 may increase the set motions by playing and reversely playing some sections of the first set section S and some sections of the second set section S.

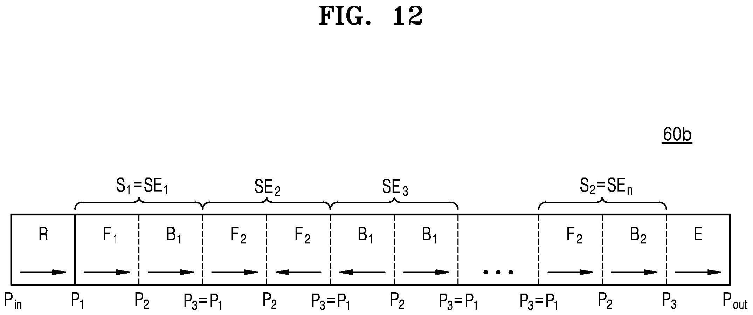

FIG. 12 is a reference diagram for describing a method of providing an exercise guide video 60b by using a plurality of set sections, according to an embodiment. The third processor 360 may increase set sections by playing and reversely playing some sections of a first set section S₁ and some sections of a second set section S₂.

As illustrated in FIG. 12, the third processor 360 may generate a first set section SE₁ by playing a forward direction section F₁ and a backward direction section B₁ of the first set section S₁ of the original video 60.

The third processor 360 may generate a second set section SE₂ by playing and reversely playing a forward direction section F₂ of the second set section S₂, and the processor 360 may generate a third set section SE₃ by reversely playing and playing the backward direction section B₁ of the first set section S₁. The processor may generate an n-1^{th} set section from a fourth set section by repeatedly performing the playing and the reverse playing of the forward direction section F₂ of the second set section S₂ and the reverse playing and the playing of the backward direction section B₁ of the first set section S₁.

The third processor 360 may generate an n^{th} set section SEₙ by sequentially playing the forward direction section F₂ and a backward direction section B₂ of the second set section S₂.

The exercise guide videos 60a and 60b may be generated by using the original video 60 including the set motions of the basic number of times, and thus, the number of times of reverse playing or the reverse playing section may be variously modified.

The original video may include one type of set motions. However, the original video is not limited thereto. The original video may include a plurality of different types of set motions. For example, the original video may include a first type set motion allowing repeated exercises in a right direction and a second type set motion allowing repeated exercises in a left direction. The third processor 360 may provide an exercise guide video by using reverse playing of some sections of the original video.

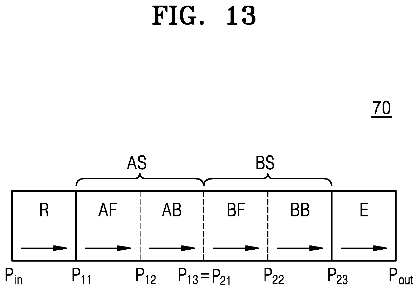

FIG. 13 is a diagram of an original video 70 including different types of set motions, according to an embodiment. Referring to FIG. 13, the original video 70 may be divided into a plurality of sections R, S, and E. The original video 70 may be divided into a preparation section R indicating a preparation motion, a finishing section E indicating a finishing motion, and a set section S which may indicate repetitive motions.

The set section S may be divided into a plurality of set sections AS and BS with respect to a point at which the type of repetitive exercise is changed. For example, the set section S may be divided into a first type set section AS and a second type set section BS with respect to a point P₁₃=P₂₁ at which the type of repetitive exercise is changed. For example, a lunge exercise may be divided into a right side lunge of a right leg at the back, that is, the first type set section AS, and a left side lunge of a left leg at the back, that is, the second type set section BS.

Each of the set sections AS and BS may be divided into a forward direction section AF or BF and a backward direction section AB or BB with respect to a point at which a direction of the motion is switched. For example, each of the right side lunge exercise and the left side lunge exercise may be divided into the forward direction section indicating a sitting down motion and the backward direction section indicating a standing up motion.

For example, the set section S of the lunge exercise may be divided into: the forward direction section AF of the first type set section AS indicating a motion from a start point P₁ of the set motion to a point P₁₂ at which the right knee forms 90 degrees and an upper body is lowered; the backward direction section AB of the first type set section AS indicating a motion from the point P₁₂ at which the right knee forms 90 degrees and the upper body is lowered to a point P₁₃=P₂₁ at which both knees get flat and the upper body is raised; the forward direction section BF of the second type set section BS indicating a motion from the point P₁₃=P₂₁ at which the both knees get flat and the upper body is raised to a point P₂₂ at which the left knee forms 90 degrees and the upper body is lowered; and the backward direction section BB of the second type set section BS indicating a motion from the point P₂₂ at which the left knee forms 90 degrees and the upper body is lowered to a point P₂₃ at which the knees get flat and the upper body is raised.

A time in which the forward direction section of each type set section and a time in which the backward direction section of each type set section corresponding thereto may be the same as each other. However, for example, the time in which the forward direction section is played and the time in which the backward direction section is played may have a difference of an error range within 10 %.

A posture in the video at the start point of each type set section and a posture in the video at the end point of each type set section corresponding thereto may be the same as each other. However, it is not limited thereto. For example, the posture at the start point of each type set section and the posture at the end point of each type set section corresponding thereto may have a difference of an error range of 10 %.

The third processor 360 may generate an exercise guide video including a plurality of set motions by using the original video 70 of FIG. 13.

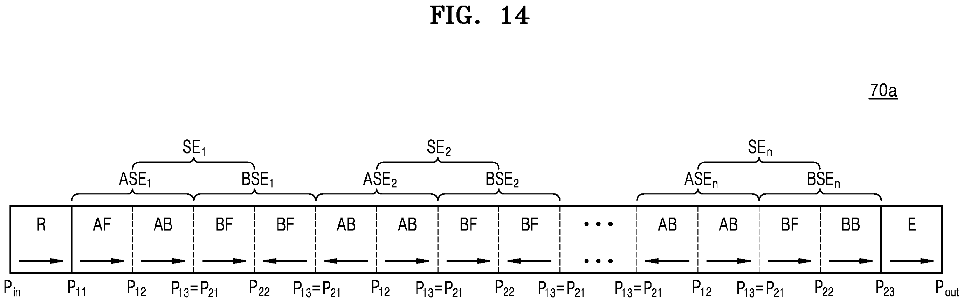

FIG. 14 is a reference diagram for describing a method of generating an exercise guide video 70a including a repetitive exercise, according to an embodiment.

When n times set motions are requested from the third user interface 320 or an external device, the third processor 360 may generate the exercise guide video 70a including n set motions based on reverse playing of some sections of the original video 70.

As illustrated in FIG. 14, the third processor 360 may play the preparation section R of the original video 70.

The third processor 360 may generate first set sections ASE₁ and BSE₁ by sequentially playing the forward direction section AF of the first type set section AS, the backward direction section AB of the first type set section AS, and the forward direction section AF of the second type set section AS and reversely playing the forward direction section BF of the second type set section BS.

When the original video includes a lunge exercise, the third processor 360 may generate a video of a right side lunge exercise by playing the forward direction section AF of the firs type set section AS and the backward direction section AB of the first type set section AS. Also, the third processor 360 may generate a video of a left side lunge exercise by playing the forward direction section BF of the second type set section BS and then reversely playing the forward direction section BF of the second type set section BS. That is, the forward direction section BF of the second type set section BS is a video indicating the motion in which the left knee is bent and the upper body is lowered, and thus, by reversely playing the forward direction section BF of the second type set section BS, a video of the motion in which the left knee gets flat and the upper body is raised may be generated.

The third processor 360 may generate second set sections ASE₂ and BSE₂ by reversely playing the backward direction section AB of the first type set section AS, sequentially playing the backward direction section AB of the first type set section AS and the forward direction section BF of the second type set section BS, and then, reversely playing the forward direction section BF of the second type set section BS.

The third processor 360 may generate n-2 set sections by performing, n-2 times, the reverse playing of the backward direction section AB of the first type set section AS, the sequential playing of the backward direction section AB of the first type set section AS and the forward direction section BF of the second type set section BS, and then, reverse playing of the forward direction section BF of the second type set section BS.

Also, the third processor 360 may generate n^{th} set sections ASEₙ and BSEₙ by reversely playing the backward direction section AB of the first type set section AS and sequentially playing the backward direction section AB of the first type set section AS, the forward direction section BF of the second type set section BS, and the backward direction section BB of the second type set section BS. Lastly, the third processor 360 may complete the generation of the exercise guide video 70a by playing the finishing section E.

FIG. 14 illustrates the method of alternately playing the first type set section AS and the second type set section BS. However, the method of generating the exercise guide video is not limited thereto. The third processor 360 may generate the first type set section n times by using the first type set section AS and may generate the second type set section n times by using the second type set section BS.

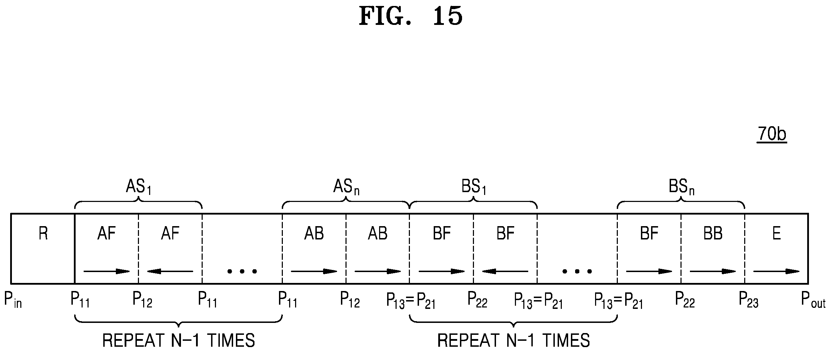

FIG. 15 is a reference diagram for describing a method of generating an exercise guide video 70b including a repetitive exercise, according to another embodiment.

When n times set motions are requested from the third user interface 310 or an external device, the third processor 360 may play the exercise guide video 70b including n set motions based on reverse playing of some sections of the original video 70.

As illustrated in FIG. 15, the third processor 360 may play the preparation section R of the original video 70.

The third processor 360 may generate n-1 times first type set sections AS₁ and AS₂ by performing n-1 times playing of the forward direction section AF of the first type set section AS and then reverse playing of the forward direction section AF of the first type set section AS. Also, the third processor 360 may generate an n^{th} first type set section ASₙ by sequentially playing the forward direction section AF of the first type set section AS and the backward direction section AB of the first type set section AS.

Also, the third processor 360 may generate n-1 times second type set sections BS₁ and BS₂ by performing, n-1 times, playing of the forward direction section BF of the second type set section BS and then reverse playing of the forward direction section BF of the second type set section BS. Also, the third processor 360 may generate an n^{th} second type set sections BSₙ by sequentially playing the forward direction section BF of the second type set section BS and the backward direction section BB of the second type set section BS.

Lastly, the third processor 360 may complete the generation of the exercise guide video 70b by playing the finishing section E.

It is described with respect to FIG. 15 that each type set section may be repeatedly generated n times based on the reverse playing of FIG. 7. However, the method is not limited thereto. For the reverse playing of the type set section, the reverse playing of FIG. 8 may be implemented. Alternatively, the reverse playing of FIG. 7 and the reverse playing of FIG. 8 may be implemented in combination.

With reference to FIGS. 14 and 15, the method of providing the exercise guide video by using the original video including two different types of set motions is described. However, the present disclosure is not limited thereto. The exercise guide video may also be provided by using the original video including three or more different types of set motions. Also, the exercise guide video may also be generated by using not only the original video including a set of different types of repetitive exercises, but also the original video including two or more basic sets of different types of repetitive exercises.

The generated exercise guide video may be provided by the mirror display 30 or may be provided by other devices.

The exercise guide video according to an embodiment may be adjusted or ended in response to an operation of a user.

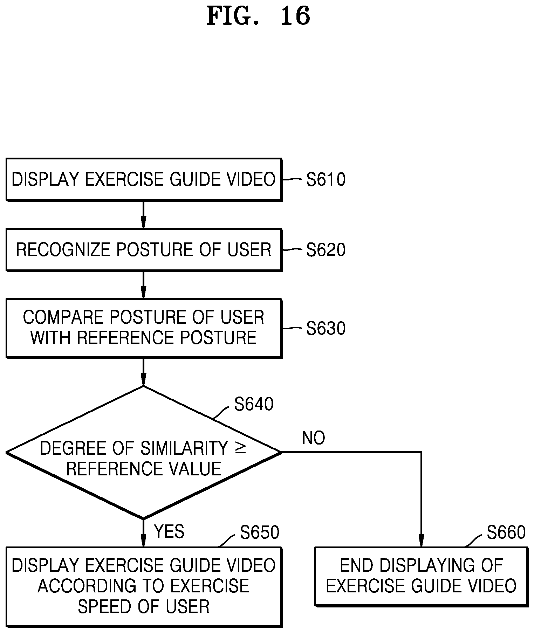

FIG. 16 is a flowchart of a method of providing an exercise guide video based on a user's motion, according to an embodiment. Referring to FIG. 16, the third processor 360 may display the exercise guide video on the display 340 in operation S610. When a user enters a predetermined distance from the mirror display 30, the third processor 360 may be switched from a stand-by mode to an operation mode. Also, the mirror display 30 may request an exercise item to be played from the user terminal 40 carried by the user, etc. The user terminal 40 may transmit a pre-stored exercise item and the number of times of a set motion of the exercise item to the mirror display 30 and may request the mirror display 30 to play an exercise guide video with respect to the corresponding exercise item.

Alternatively, the user may input the exercise item and the number of times of the set motion through the third user interface 330 of the mirror display 30 in order to input a command for displaying the exercise guide video.

The third processor 360 may generate the exercise guide video including a plurality of set motions based on reverse playing of some sections of an original video as described above and may display the generated exercise guide video on the display 340. However, it is not limited thereto. The second memory 320 may pre-store the exercise guide video for each user or each set. The third processor 360 may display the exercise guide video corresponding to the pre-stored user or set on the display 340 by reading the exercise guide video according to a command for requesting the playing thereof.

The third processor 360 may recognize a posture of the user in operation S620. The motion sensing sensor 370 may transmit a result of sensing a motion of the user to the third processor 360. The motion sensing sensor 370 may be equipment for obtaining a user's motion and may include a vision camera, a vision sensor, an acceleration sensor, a gyro sensor, a geomagnetic sensor, an infrared sensor, etc. The third processor 360 may recognize the posture of the user by using motion data received from the motion sensing sensor 370.

For example, when the motion data is an image including the user, the third processor 360 may extract, from the image including the user, feature points for recognizing the posture. The feature point may be an area indicating a body part of a human being. For example, the feature point may be the shoulder, the shoulder joint, the upper arm, the elbow joint, the lower arm, the wrist joint, the hand, the body, the coxa joint, the upper leg, the knee joint, the lower leg, the ankle joint, the foot, etc. The third processor 360 may recognize the posture of the user by analyzing the feature points, a connection angle (a three-dimensional angle) of each feature point, a distance (a three-dimensional distance) of each feature point, etc.

For example, the third processor 360 may recognize a two-dimensional (2D) posture by using a deep learning method, a fast human pose estimation (FPD) learning method, etc. for the motion data. Alternatively, the third processor 360 may recognize a three-dimensional (3D) posture by using a model-based method using a 3D body model, a model-free method for the estimation without a 3D model, or the like. However, it is not limited thereto. Various known techniques for recognizing the posture of the human body may be used.

The third processor 360 may recognize a posture of a target (for example, a trainer) included in the exercise guide video (hereinafter, referred to as a "reference posture) by using substantially the same method as the method of recognizing the posture of the user. However, it is not limited thereto. The third processor 360 may generate motion modeling by estimating the reference posture from the original video and may pre-store the original video together with the motion modeling. Also, when the third processor 360 displays the exercise guide video based on the original video, the third processor 360 may extract the reference posture by also changing the motion modeling matching the displayed exercise guide video. The motion modeling may be performed by the third processor 360, but may also be performed by an external device, such as a server.

Alternatively, when the motion sensing sensor 370 is a vision camera configured to photograph a posture of the user and a posture of the target displayed on a screen of the display 340, the third processor 360 may recognize the posture of the user and the reference posture by using the method of extracting the posture of the user and the reference posture from the sensed image data.

The third processor 360 may compare the posture of the user with the reference posture corresponding to the posture of the user and included in the exercise guide video in operation S630. The third processor 360 may calculate a degree of similarity between a posture of the user at a predetermined time point, for example, a current posture of the user, and the reference posture included in the exercise guide video and corresponding to the posture of the user.

The posture of the user recognized at a predetermined time point and the reference posture displayed on the display 340 may be different from each other. For example, when an exercise speed of the user is less than a play speed of the exercise guide video, the posture of the user may lag behind the reference posture displayed on the display 340. That is, the reference posture corresponding to the posture of the user included in the exercise guide video may have been displayed already. The third processor 360 may compare the recognized posture of the user with the pre-displayed reference posture corresponding to the posture of the user.

Alternatively, when the exercise speed of the user is greater than the play speed of the exercise guide video, the posture of the user may get ahead of the reference posture displayed on the display 340. That is, the reference posture corresponding to the posture of the user included in the exercise guide video may not have been displayed yet. The third processor 360 may compare the recognized posture of the user with the reference posture corresponding to the posture of the user, only when the reference posture corresponding to the posture of the user is displayed on the display 340.

A higher degree of similarity denotes a higher matching rate between the posture of the user and the reference posture. The third processor 360 may use the degree of similarity based on angles of the feature points or distances between the feature points. The third processor 360 may calculate the degree of similarity based on the angles of the feature points or the distances between the feature points and may quantitatively calculate the degree of similarity by summing weights respectively assigned to the similarities. The degree of similarity may be calculated for all of the feature points. However, the degree of similarity may be calculated for one or more important feature points in the reference posture. As the method of calculating the degree of similarity, various known methods may be used, and thus, the detailed description thereof is omitted.

When the degree of similarity between the posture of the user and the reference posture is greater than or equal to a reference value (S640-Yes), the third processor 360 may display the exercise guide video on the display 340 according to the exercise speed of the user, in operation S650.

The play speed of the exercise guide video may be determined based on the original video. Alternatively, the play speed of the exercise guide video may be adjusted according to a user's command, etc. However, the play speed may be fixed regardless of the exercise speed of the user, and thus, the play speed of the exercise guide video and the exercise speed of the user may be different from each other.

When the user is elderly or is not in a good condition, or when the user is a novice exerciser, the exercise speed of the user may be less than the play speed of the exercise guide video. When the user performs an exercise according to the play speed of the exercise guide video, the user may get hurt or may not be able to perform the exercise with the correct posture. Alternatively, as the number of times of the set motion increases, the exercise speed of the user may decrease, while the play speed of the exercise guide video may be constant. Thus, the user may get hurt or may not be able to perform the exercise with the correct posture.

The third processor 360 according to an embodiment may adjust the play speed of the exercise guide video according to the exercise speed of the user.

When it is determined that the posture of the user is ahead of the reference posture corresponding to the posture of the user by a reference section, the third processor 360 may determine that the exercise speed of the user may be greater than the play speed of the exercise guide video. Here, the reference section may be a time section corresponding to a sub-set motion of the set motion or the set motion. For example, in the case of a squat exercise, each of a forward direction section and a backward direction section may be the time section corresponding to the sub-set motion, and the sum of the forward direction section and the backward direction section may be the time section corresponding to the set motion. The reference section may be differently pre-set by a user, a manager, etc. according to an exercise item.

The third processor 360 may determine a currently recognized posture of the user based on a previously recognized posture of the user. For example, the third processor 360 may determine whether the currently recognized posture of the user is a posture of the forward direction section or the backward direction section of the squat exercise based on the previously recognized posture of the user. Likewise, the third processor 360 may determine a current reference posture based on a previous reference posture.

The third processor 360 may increase the play speed of the exercise guide video such that the displayed reference posture may correspond to the recognized posture of the user. The play speed may be proportionately adjusted according to the exercise speed of the user or may be adjusted to have a step-by-step difference. For example, the third processor 360 may adjust the play speed of the exercise guide video to be any one of 1.1 times, 1.2 times, 1.5 times, and 2 times such that the displayed reference posture may correspond to the posture of the user, that is, the reference posture displayed at a predetermined time point may correspond to the posture of the user.

When it is determined that the posture of the user is behind the displayed reference posture by a reference section, the third processor 360 may determine that the exercise speed of the user may be less than the play speed of the exercise guide video. Thus, the third processor 360 may decrease the play speed of the exercise guide video so that the displayed reference posture may correspond to the recognized posture of the user, that is, the reference posture displayed at a predetermined time point may correspond to the posture of the user. The play speed may be proportionately adjusted according to the motion speed of the user or may be adjusted to have a step-by-step difference. For example, the third processor 360 may adjust the play speed of the exercise guide video to be any one of 0.9 times, 0.8 times, and 0.5 times such that the reference posture may correspond to the posture of the user.

When it is determined that the posture of the user is behind the reference posture by the reference section, the third processor 360 may pause the playing of the exercise guide video until the displayed reference posture may correspond to the posture of the user. Also, when the posture of the user and the reference posture displayed on the exercise guide video correspond to each other, the third processor 360 may resume the playing of the exercise guide video.

The third processor 360 is described to determine the relationship between the exercise speed of the user and the play speed based on the recognized posture of the user and the reference posture. However, the third processor 360 is not limited thereto. The third processor 360 may determine the relationship between the exercise speed of the user and the play speed based on a time in which a motion of a predetermined user is performed and a playing time in which a reference motion corresponding to the motion of the predetermined user is displayed.

For example, the third processor 360 may profile the movement of the user, that is, the motion of the user, from the recognized postures of the user and may divide the profiled motion of the user into sub-set units. Also, the third processor may compare a time taken for the user to perform the sub-sets with a playing time taken to play sub-set sections of the exercise guide video.

When a difference between the motion time and the playing time is within a reference time, the third processor may determine that the exercise speed of the user and the play speed are the same as each other. When the motion time is greater than the playing time by the reference time or greater, the third processor may determine that the exercise speed of the user is less than the play speed. Alternatively, when the motion time is less than the playing time by the reference time or greater, the third processor may determine that the exercise speed of the user is greater than the play speed. Here, the reference time may be a time corresponding to the sub-set section and may be differently set by a user, a manager, etc. according to an exercise item. In addition, as the method of calculating the exercise speed of the user, a method of calculating the exercise speed according to the related art may be used.

When the degree of similarity between the posture of the user and the reference posture corresponding to the posture of the user is less than a reference value during a predetermined time period, the third processor 360 may end displaying of the exercise guide video. The predetermined time period may correspond to a one-time set motion. However, it is not limited thereto. The predetermined time period may correspond to two or three times set motions.

When a result with respect to the motion of the user is not received from the motion sensing sensor 370 during a predetermined time period, the third processor 360 may end the displaying of the exercise guide video. When the result with respect to the motion of the user is not received from the motion sensing sensor 370, the third processor 360 may determine the degree of similarity between the posture of the user and the reference posture as "0." When the motion sensing sensor 370 is not able to detect the motion of the user, it may denote that the user deviates from the exercise place.

When the posture of the user that does not correspond to the reference posture continues during a predetermined time period, the third processor 360 may end the displaying of the exercise guide video to prevent unnecessary playing of the exercise guide video. Thus, power consumption of an exercise machine or the mirror display 30 may be saved.

While the third processor 360 is providing the exercise guide video, the third processor 360 may provide information about the play speed of the exercise guide video and information about the degree of similarity between the posture of the user and the reference posture. The information above may be provided by at least one of a character, a graph, text, and sound.

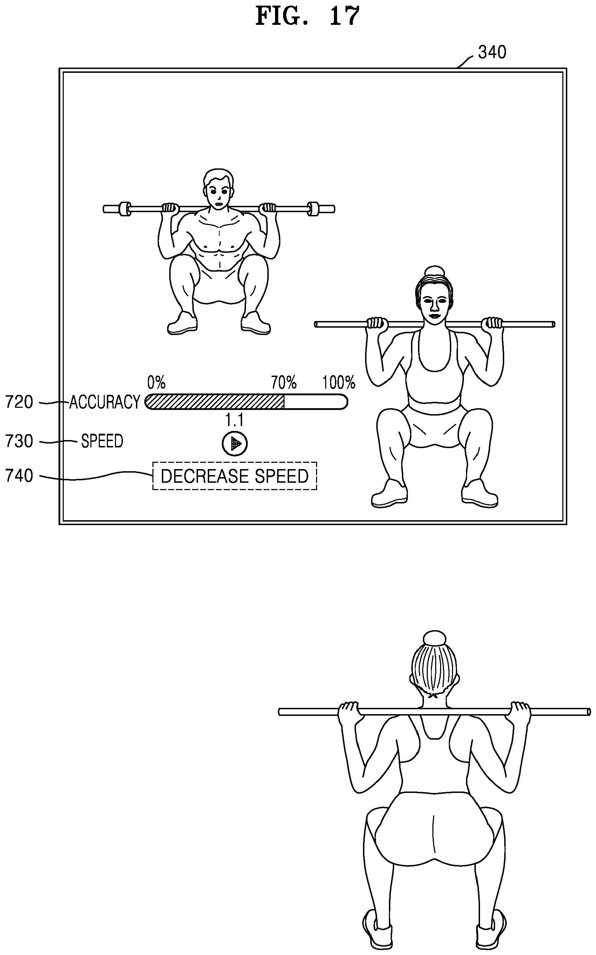

FIGS. 17 to 19 are reference diagrams for describing a method of providing an exercise guide video, according to an embodiment.

As illustrated in FIG. 17, the third processor 360 may display an exercise guide video 710 on the display 340. A user may perform an exercise watching a reference posture displayed on the exercise guide video. The third processor 360 may compare a posture of the user with a reference posture corresponding to the posture of the user and calculate a degree of similarity between the posture of the user and the reference posture and then may display a result 720 thereof on the display 340. The user may identify the accuracy of his/her posture based on the similarity.

Also, when it is determined that the posture of the user is ahead of the reference posture corresponding to the posture of the user by a reference section, the third processor 360 may determine that an exercise speed of the user is greater than a play speed of the exercise guide video. The third processor 360 may increase the play speed of the exercise guide video so that the displayed reference posture corresponds to the posture of the user.

Also, as illustrated in FIG. 17, the third processor 360 may display a first indicator 730 indicating that the play speed of the exercise guide video is increased on the display 340. In addition, the third processor 360 may display a second indicator 740 for guiding the user to lower the exercise speed on the display 340.

Alternatively, when it is determined that the posture of the user is behind the reference posture by a reference section, the third processor 360 may determine that the exercise speed of the user is less than the play speed of the exercise guide video. Also, the third processor 360 may decrease the play speed of the exercise guide video so that the displayed reference posture corresponds to the posture of the user.

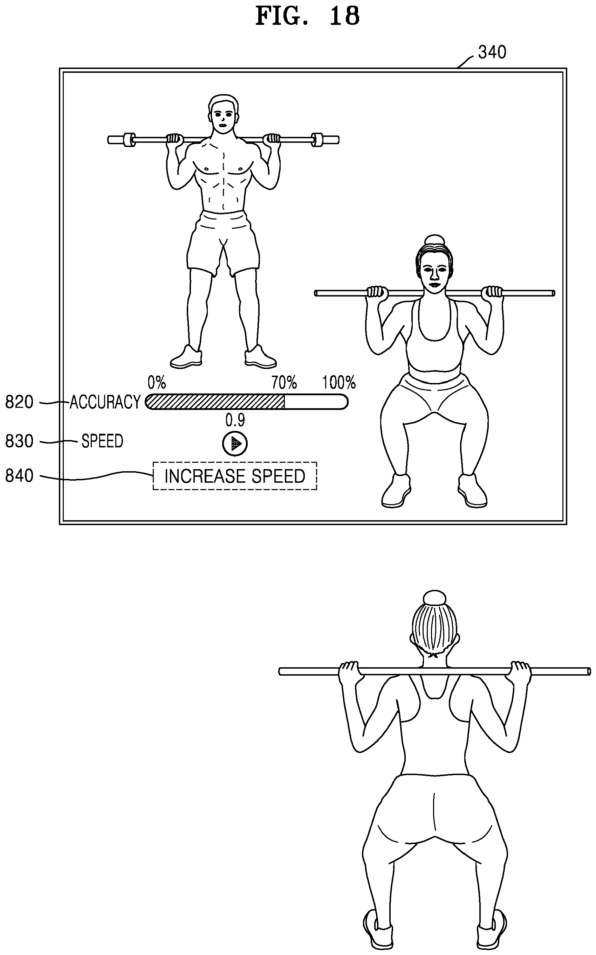

Also, as illustrated in FIG. 18, the third processor 360 may display a third indicator 830 indicating that the play speed of the exercise guide video is decreased on the display 340. In addition, the third processor 360 may display a fourth indicator 840 for guiding the user to raise the exercise speed on the display 340.

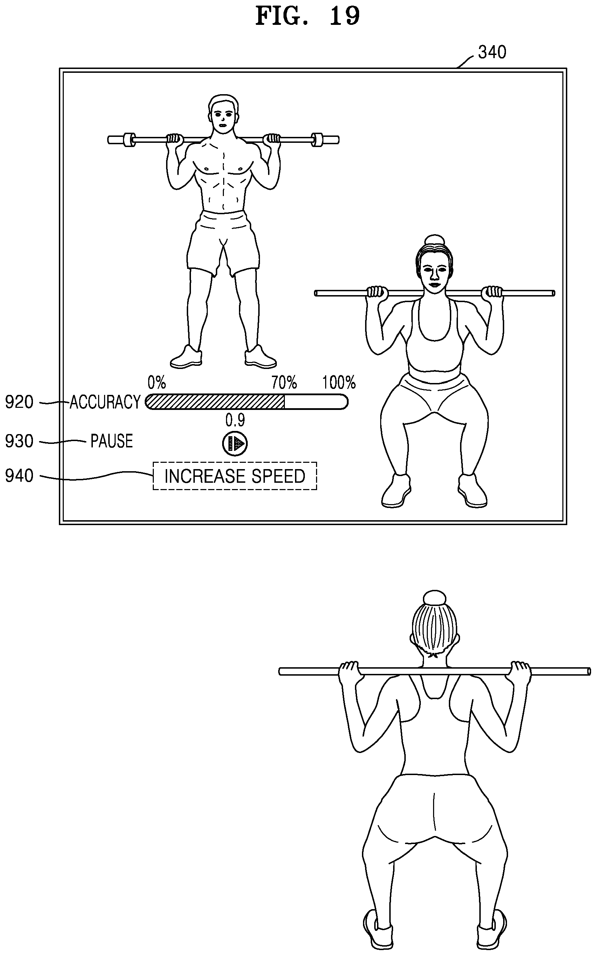

Alternatively, as illustrated in FIG. 19, when it is determined that the posture of the user is behind the reference posture corresponding thereto by the reference section, the third processor 360 may pause the playing of the exercise guide video until the displayed reference posture corresponds to the posture of the user. Also, as illustrated in FIG. 19, the third processor 360 may display a fifth indicator 930 indicating that the playing of the exercise guide video is paused on the display 340. When the posture of the user gets to resemble the reference posture displayed on the display 340, the third processor 360 may resume the playing of the exercise guide video.

Right at the moment at which it is determined that the posture of the user is behind the reference posture by the reference section, the third processor 360 may pause the playing of the exercise guide video. However, it is not limited thereto. When it is determined that the posture of the user is behind the reference posture by the reference section, the third processor 360 may pause the playing of the exercise guide video at a point of the exercise guide video, at which a sub-set motion or a set motion is ended.

The third processor 360 may store the exercise speed of the user and may refer to the stored exercise speed when the third processor 360 subsequently plays the exercise guide video. For example, when the user exercises by a greater speed than the play speed every time the user exercises, the third processor 360 may increase the play speed of the exercise guide video to be greater than a pre-set play speed.

It is described that the third processor 360 may provide the indicator indicating the degree of similarity between the posture of the user and the reference posture corresponding thereto. However, the third processor 360 is not limited thereto. The third processor 360 may also provide information about an effect of the exercise of the user. The third processor 360 may display the exercise effect by using a body shape of a character referring to the user, for example, an avatar.

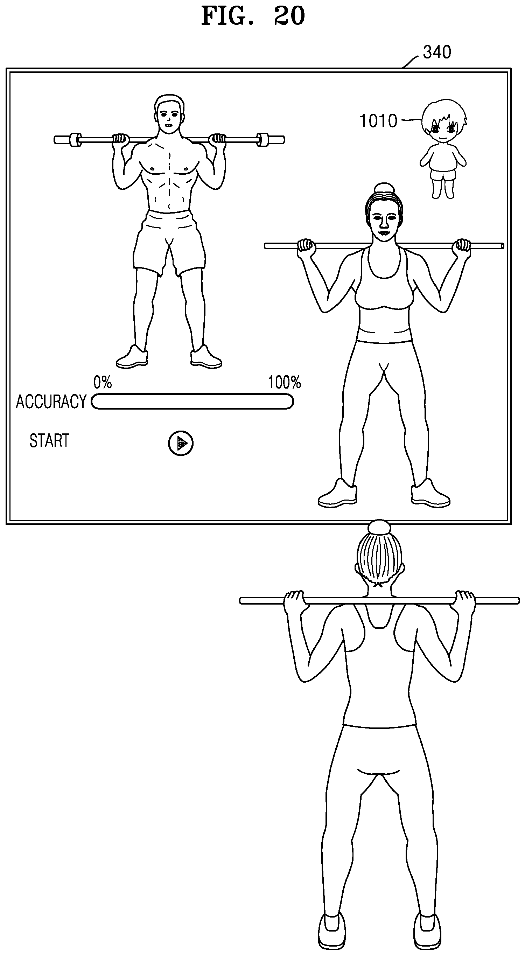

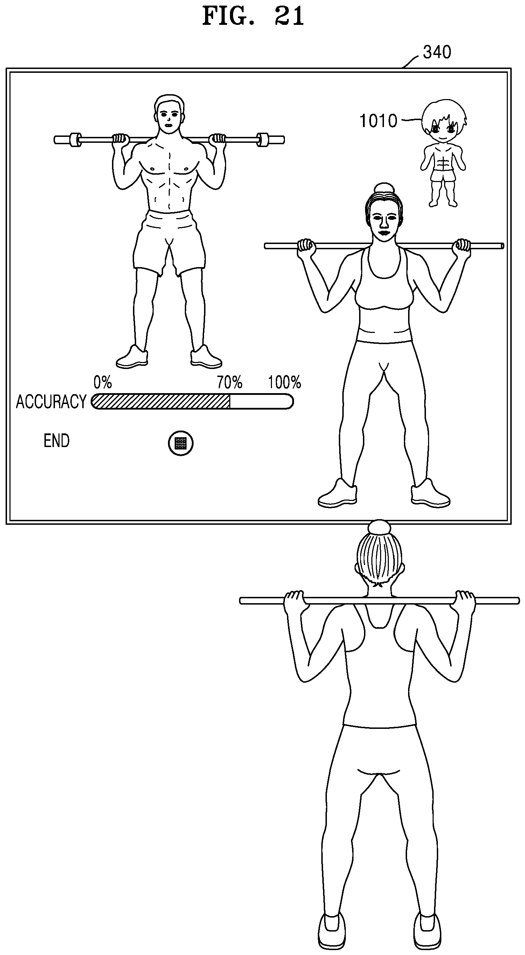

FIGS. 20 and 21 are reference diagrams for describing a method of providing an exercise effect according to an embodiment. As illustrated in FIG. 20, the third processor 360 may display a character 1010 referring to a user before playing an exercise guide video.

Also, while the third processor 360 is displaying the exercise guide video, the third processor 360 may compare a posture of the user with a reference posture corresponding to the posture of the user and calculate a degree of similarity between the posture of the user and the reference posture. The third processor 360 may change the body shape of the character based on a result of the calculated similarity and may display the character 1010 of the changed body shape on the display 340. For example, because a squat exercise has the effect of strengthening the lower body muscles and reducing fats, the third processor 360 may display the character 1010 having strengthened lower body muscles and reduced fats on the display 340, as illustrated in FIG. 21.

It is described above that the mirror display 30 may generate the exercise guide video. However, the present disclosure is not limited thereto. The exercise guide video may be generated by an exercise machine or generated by a server capable of communication with the exercise machine or the mirror display 30. Also, the exercise machine or the mirror display 30 may display the generated exercise guide video. In addition, the exercise guide video may be generated by general electronic devices including displays, for example, a smartphone, a PC, a tablet PC, a notebook computer, a smart TV, a cellular phone, a PDA, a laptop computer, a media player, a digital broadcasting terminal, a navigation device, a wearable device and other mobile devices, immobile computing devices, etc. and may also be generated by an electronic device capable of communicating with a display device.

The method according to an embodiment of the present disclosure may be implemented in the form of a program command executable by various computer devices and may be recorded on a computer-readable medium. The computer-readable medium may include a program command, a data file, a data structure, etc. individually or in a combined fashion. The program command recorded on the medium may be specially designed and configured for the present disclosure or may be well known to and usable by one of ordinary skill in the art. Examples of the computer-readable recording medium include magnetic media (e.g., hard discs, floppy discs, or magnetic tapes), optical media (e.g., compact disc-read only memories (CD-ROMs), or digital versatile discs (DVDs)), magneto-optical media (e.g., floptical discs), and hardware devices that are specially configured to store and carry out program commands (e.g., ROMs, random-access memories (RAMs), or flash memories). Examples of the program commands include a high-level language code executable by a computer by using an interpreter, etc., as well as a machine language code, such as the one made by a complier.

One or more embodiments of the present disclosure may also be realized in the form of a recording medium including instructions executable by a computer, such as a program module executed by a computer. The computer-readable recording medium may be an arbitrary available medium accessible by a computer and includes all of volatile and non-volatile media and detachable and non-detachable media. Also, the computer-readable recording medium may include both of a computer storage medium and a communication medium. The computer storage recording medium includes all of volatile and non-volatile media and detachable and non-detachable media that are realized by an arbitrary method or technique for storing information, such as computer-readable instructions, data structures, program modules, or other data. The communication medium typically includes computer-readable instructions, data structures, program modules, or other data of modulated data signals, such as carrier waves, or other transmission mechanisms, and includes an arbitrary data transmission mechanism. Also, an embodiment of the present disclosure may also be implemented by a computer program or a computer program product including a computer-executable instruction, such as a computer program executable by a computer.

Machine-readable storage media may be provided as non-transitory storage media. Here, the term "non-transitory storage media" only denotes that the media are tangible devices and do not include signals (e.g., electromagnetic waves), and does not distinguish the storage media semi-permanently storing data and the storage media temporarily storing data. For example, the "non-transitory storage media" may include a buffer temporarily storing data.

According to an embodiment, the method according to various embodiments disclosed in the present specification may be provided as an inclusion of a computer program product. The computer program product may be, as a product, transacted between a seller and a purchaser. The computer program product may be distributed in the form of a machine-readable storage medium (e.g., a compact disc (CD)-ROM) or may be distributed online (e.g., downloaded or uploaded) through an application store or directly between two user devices (e.g., smartphones). In the case of online distribution, at least part of a computer program product (e.g., a downloadable application) may be at least temporarily stored in a machine-readable storage medium, such as a server of a manufacturer, a server of an application store, or a memory of a relay server, or may be temporarily generated.

One or more desirable embodiments are described above. It would be understood by one of ordinary skill in the art that the present disclosure may be realized in a different form modified within a range not departing from the essential properties of the present disclosure. Therefore, the embodiments should be considered in a descriptive sense only and not for purposes of limitation. The scope of the present disclosure is defined not by the detailed description of the present disclosure but by the appended claims, and all differences within the scope will be construed as being included in the present disclosure.

## Claims

1. A method of providing an exercise guide video, the method comprising:
displaying an exercise guide video including repetitive motions;
recognizing a posture of a user while the exercise guide video is being displayed;
comparing the posture of the user with a reference posture included in the exercise guide video and corresponding to the posture of the user; and
when, during a certain time period, a degree of similarity between the posture of the user and the reference posture is less than a reference value, ending the displaying of the exercise guide video.

2. The method of claim 1,
wherein the certain time period is greater than or equal to a time period corresponding to one set motion included in the exercise guide video.

3. The method of claim 1,
further comprising, when the degree of similarity between the posture of the user and the reference posture is greater than or equal to the reference value, displaying the exercise guide video according to an exercise speed of the user.

4. The method of claim 3,
wherein the displaying of the exercise guide video includes, when it is determined that the posture of the user is ahead of the reference posture displayed in the exercise guide video by a reference section, increasing a play speed of the exercise guide video such that the displayed reference posture corresponds to the posture of the user.

5. The method of claim 3,
wherein the displaying of the exercise guide video includes, when it is determined that the posture of the user is behind the reference posture displayed in the exercise guide video by a reference section, decreasing a play speed of the exercise guide video such that the displayed reference posture corresponds to the posture of the user.

6. The method of claim 3,
wherein the displaying of the exercise guide video includes, when it is determined that the posture of the user is behind the reference posture displayed in the exercise guide video by a reference section, pausing the displaying of the exercise guide video until the displayed reference posture corresponds to the posture of the user.

7. The method of claim 3,
wherein a reference section is greater than or equal to a section corresponding to a sub-set motion in the exercise guide video.

8. The method of claim 1,
further comprising:
when, during the certain time period, the degree of similarity between the posture of the user and the reference posture is greater than or equal to the reference value, changing, according to the degree of similarity, a body shape of a character indicating the user; and
displaying the changed body shape of the character.

9. The method of claim 1,
further comprising generating the exercise guide video including two or more set motions, based on reverse playing of some sections of an original video including one set motion.

10. A computer-readable recording medium having recorded thereon a program for executing, on a computer, the method of any one of claims 1 to 9.

11. An electronic device comprising:
a display configured to display an exercise guide video including repetitive motions;
a motion sensing sensor configured to recognize a posture of a user;
and
a processor configured to compare the posture of the user with a reference posture included in the exercise guide video and corresponding to the posture of the user and, when, during a certain time period, a degree of similarity between the posture of the user and the reference posture is less than a reference value, end the displaying of the exercise guide video.

12. The electronic device of claim 11,
further comprising a film disposed on the display and configured to transmit light corresponding to the exercise guide video and reflect light corresponding to the posture of the user.

13. The electronic device of claim 11,
wherein the certain time period is greater than or equal to a time period corresponding to one set motion included in the exercise guide video.

14. The electronic device of claim 11,
wherein the processor is further configured to, when the degree of similarity between the posture of the user and the reference posture is greater than or equal to the reference value, display the exercise guide video according to an exercise speed of the user.

15. The electronic device of claim 14,
wherein the processor is further configured to, when it is determined that the posture of the user is ahead of the reference posture displayed in the exercise guide video by a reference section, increase a play speed of the exercise guide video such that the displayed reference posture corresponds to the posture of the user.
